(19) Europäisches Patentamt / European Patent Office / Office européen des brevets

(11) EP 2 257 635 B1

(12) **EUROPÄISCHE PATENTSCHRIFT**

(45) Veröffentlichungstag und Bekanntmachung des Hinweises auf die Patenterteilung:
**22.02.2012 Patentblatt 2012/08**

(21) Anmeldenummer: **09727980.6**

(22) Anmeldetag: **26.03.2009**

(51) Int Cl.:
***C12P 7/02*** *(2006.01)* ***C12P 41/00*** *(2006.01)*
***C12N 9/04*** *(2006.01)*

(86) Internationale Anmeldenummer:
**PCT/EP2009/053576**

(87) Internationale Veröffentlichungsnummer:
**WO 2009/121785 (08.10.2009 Gazette 2009/41)**

# (54) VERFAHREN ZUR ENZYMATISCHEN DERACEMISIERUNG VON SEKUNDÄREN ALKOHOLEN

PROCESS FOR THE ENZYMATIC DERACEMIZATION OF SECONDARY ALCOHOLS

PROCÉDÉ DE DÉRACÉMISATION ENZYMATIQUE D'ALCOOLS SECONDAIRES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **01.04.2008 AT 5032008**

(43) Veröffentlichungstag der Anmeldung:
**08.12.2010 Patentblatt 2010/49**

(73) Patentinhaber: **Evonik Degussa GmbH**
**45128 Essen (DE)**

(72) Erfinder:
- **DINGERDISSEN, Uwe**
  **64342 Seeheim (DE)**
- **PFEFFER, Jan**
  **45355 Essen (DE)**
- **KROUTIL, Wolfgang**
  **A-8010 Graz (AT)**
- **GRUBER, Christian**
  **A-8010 Graz (AT)**
- **VOSS, Constance**
  **A-8010 Graz (AT)**

(56) Entgegenhaltungen:
**EP-A1- 1 854 893**

- **VOSS, C.V. ET AL.: "Deracemization of Secondary Alcohols through a Concurrent Tandem Biocatalytic Oxidation and Reduction" ANGEWANDTE CHEMIE INTERNATIONAL EDITION, Bd. 47, Nr. 4, 11. Januar 2008 (2008-01-11), Seiten 741-745, XP002577011 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Gebiet der Erfindung

Die vorliegende Erfindung betrifft ein Verfahren zur Deracemisierung von Enantiomerengemischen unter Verwendung von Enzymsystemen.

Stand der Technik

Auf dem Gebiet der Stereoisomerie wurden in letzter Zeit beträchtliche Fortschritte bei der Racemisierung, d.h. Umwandlung eines optischen Isomers in sein Gegenstück, um ein racemisches Gemisch zu erhalten, und Deracemisierung, der exakten Umkehr dieses Vorgangs, erzielt. Während eine Racemisierung bei stereolabilen Verbindungen wie etwa Cyanhydrinen Halb(thio)acetalen $\alpha$-substituierten Carbonylverbindungen und $\alpha$-substituierten Hydantoinen durch einfache, schonende Säure- oder Basenkatalyse erzielbar ist, sind stereostabile Verbindungen, wie z.B. sekundäre Alkohole und chirale Amine, weitaus schwieriger zu racemisieren.

Letzteres gelang beispielsweise mittels übergangsmetallkomplexkatalysierter Redoxprozesse, in denen ein Enantiomer, das am Chiralitätszentrum naturgemäß $sp^3$-hybrisiert ist, über ein prochirales, $sp^2$-hybrisiertes Zwischenprodukt in das andere übergeführt wird. Siehe z.B. die Arbeiten von O. Pamies und J.-E. Bäckvall, Trends Biotechnol. 22, 130-135 (2004) und Chem. Rev. 103, 3247-3261 (2003); H. Pellissier, Tetrahedron 59, 8291-8327 (2003); M. J. Kim, Y. Ahn und J. Park, Curr. Opin. Biotechnol. 13, 578-587 (2002); V. Zimmermann, M. Beller und U. Kragl, Org. Process Res. Dev. 10, 622-627 (2006); Y. Asano und S. Yamaguchi, J. Am. Chem. Soc. 127, 7696-7697 (2005).

Auf dem Gebiet der - inhärent hochspezifischen - Biosynthese sind nur wenige "echte" Racemasen bekannt, da in der Natur - zum Unterschied zur Industrie - kaum Bedarf an Racemisierung besteht. So sind beispielsweise einige wenige spezielle Enzyme zur Katalyse der Racemisierung von $\alpha$-Hydroxycarbonsäuren (wie z.B. Mandelsäure-Derivaten), $\alpha$-Aminosäuren und Hydantoinen bekannt (siehe z.B. B. Schnell, K. Faber und W. Kroutil, Adv. Synth. Catal. 345, 653-666 (2003)). Für die Racemisierung sekundärer Alkohole und primärer Amine waren lange Zeit praktisch keine definierten Enzyme bekannt.

Die Arbeitsgruppe der vorliegenden Erfinder offenbarte in Chem. Eur. J. 13, 8271-8276 (2007), eine neue Racemisierungsstrategie, der statt einer kinetischen eine thermodynamische Betrachtungsweise der ablaufenden Reaktionen zugrundelag: In einem Reaktionssystem, das aus den beiden Enantiomeren R und S und einem prochiralen Zwischenprodukt P besteht, steht jeder der beiden optischen Antipoden mit dem Zwischenprodukt in chemischem und thermodynamischem Gleichgewicht, d.h. $P \rightleftharpoons S$ und $R \rightleftharpoons P$. Unter Verwendung mehrerer Kombinationen aus zwei entgegengesetzt enantioselektiven Alkohol-Dehydrogenasen (in der Folge kurz ADHs genannt), die denselben Cofaktor, entweder NAD oder NADP (Nicotinamid-Adenin-Dinucleotid bzw. -Phosphat), nutzen, gelang die Racemisierung verschiedener, optisch aktiver sekundärer Alkohole, einschließlich Acyloine. Das Gleichgewicht Alkohol/Keton wird durch geeignete Wahl der Menge und des Verhältnisses zwischen oxidierter und reduzierter Form des Cofaktors, d.h. $NAD^+$: NADH bzw. $NADP^+$: NADPH auf der Seite des Alkohols gehalten, vgl. erläuternd Figur 1.

Wurde der Anteil an $NAD(P)^+$ auf ein Minimum gesetzt, fiel - ausgehend vom reinen (S)-Isomer - nach wenigen Stunden Reaktionszeit das gewünschte Racemat an. Die Menge an Keton-Zwischenprodukt konnte auf unter 10 %, zum Teil auf unter 1 % gesenkt werden. Vergleichsversuche mit nur einer hochselektiven ADH schlugen hingegen für die meisten getesteten ADHs fehl. Lediglich in einem Fall wurde nach einer Reaktionszeit von 14 Tagen eine Ausbeute von 82 % ee ("enantiomeric excess", Enantiomerenüberschuss, d.h. optische Ausbeute) erzielt.

In einer erst kürzlich publizierten Arbeit der vorliegenden Erfinder (C. V. Voss, C. C. Gruber und W. Kroutil, Angew. Chem. Int. Ed. 47, 741-745 (2008)) wird die Deracemisierung von Racematen sekundärer Alkohole über ein prochirales Keton als Zwischenprodukt unter Verwendung eines Tandem-Systems aus enantioselektiven bakteriellen Enzymen für die Alkohol-Oxidation in Form von *Alcaligenes faecalis*-Zellen, einer stereoselektiven ADH und NAD als Cofaktor offenbart. Der Cofaktor wurde gewissermaßen "regeneriert", d.h. von der oxidierten in die reduzierte Form zurückgeführt, indem parallel eine mittels Glucose-Dehydrogenase (in der Folge kurz GDH) als "Zusatz-" oder "Hilfsenzym" katalysierte Oxidation von Glucose zu Gluconolacton bzw. Gluconsäure ablaufen gelassen wurde, vgl. erläuternd Figur 2.

Bei anfänglichen Versuchen unter Verwendung von lyophilisierten *Alcaligenes* faecalis-Zellen wurde überraschenderweise keine Deracemisierung, sondern vielmehr Racemisierung von enantiomerenreinen Alkoholen als Ausgangssubstrate festgestellt, was auf eine Erhöhung der Zellpermeabilität durch die Lyophilisierung zurückgeführt wurde. Unter Einsatz frisch geernteter Zellen mit intakter Zellmembran, so dass Oxidation und Reduktion räumlich getrennt voneinander abliefen, konnten Racemate verschiedener sekundärer Alkohole selektiv und in Ausbeuten von > 99 % ee in das gewünschte Enantiomer übergeführt werden.

Dieser Stand der Technik weist allerdings mehrere Nachteile auf. Zum einen ist das *Alcaligenes faecalis*-System, das ein Enzymgemisch für die Oxidation bereitstellt, nicht exakt definierbar, so dass es zu erheblichen Variationen der Reaktionsabläufe kommen kann und somit die Reproduzierbarkeit nicht allzu hoch ist. Zum anderen werden bei allen bisherigen Verfahren pro Mol an isomerisiertem Alkohol 1 Mol Sauerstoff für die Oxidation und stöchiometrisch 1 Mol Glucose für die Cofaktor-Regeneration verbraucht, wobei zudem 1 Mol Gluconsäure bzw. Gluconolacton als Nebenprodukt anfällt.

Die Patentschrift EP 1 854 893 A1 (KANEKA CORPORATION, 14. November 2007) offenbart ein Verfahren zur Deracemisierung sekundärer Alkohole unter Verwendung einer Kombination zweier Alkohol-Dehydrogenasen mit entgegengesetzten Stereoselektivitäten und verschiedenen Cofaktorselektivitäten, wobei die verbrauchten Cofaktoren jeweils mit einem separaten Zusatzenzymsystem regeneriert werden.

Aufgabe der Erfindung war daher die Bereitstellung eines verbesserten Deracemisierungsverfahrens, das die obigen Nachteile vermeidet.

Beschreibung der Erfindung

Überraschenderweise wurde gefunden, dass ein verbessertes Verfahren zur enzymatischen Deracemisierung von Enantiomerengemischen sekundärer Alkohole durch eine Kombination aus Oxidations- und Reduktionsreaktionen mittels stereoselektiver Alkohol-Dehydrogenasen und derer Cofaktoren diese Aufgabe löst, wobei formal ein Enantiomer eines optisch aktiven sekundären Alkohols selektiv zum entsprechenden Keton oxidiert wird, das anschließend selektiv zum optischen Antipoden reduziert wird, während die reduzierte Form des Cofaktors für die Reduktionsreaktion über ein Zusatzenzym zur Verfügung gestellt wird. Das erfindungsgemäße Verfahren ist dadurch gekennzeichnet, dass zwei Alkohol-Dehydrogenasen mit entgegengesetzter Stereoselektivität und unterschiedlicher Cofaktorselektivität sowie die zwei zugehörigen, unterschiedlichen Cofaktoren für die Oxidations- bzw. die Reduktionsreaktion eingesetzt und die oxidierten und reduzierten Cofaktoren in einer parallel ablaufenden enzymatischen Reaktion mit dem Zusatzenzym ineinander übergeführt werden, wobei die Richtung der Deracemisierung hin zu einem der beiden Enantiomere durch die Wahl der beiden Alkohol-Dehydrogenasen bzw. anhand des Selektivitätsunterschieds des Zusatzenzyms für die beiden Cofaktoren steuerbar ist.

Durch das erfindungsgemäße Verfahren sind Deracemisierungen mit praktisch quantitativer optischer Ausbeute, d.h. > 99 % ee, erzielbar, ohne dass Reagentien im Verlauf der parallelen Reaktionen stöchiometrisch verbraucht werden, sobald das System ein stabiles Gleichgewicht erreicht hat, wie später noch näher erläutert wird. Weiters werden genau definierte, reine Enzyme (die beiden ADHs und das Zusatzenzym) zur Katalyse eingesetzt, woraus ausschließlich reversible Reaktionen im Verfahren sowie eine ausgezeichnete Reproduzierbarkeit resultieren. Und schließlich kann das Verfahren in einfachen Eintopfreaktionen durchgeführt werden, ohne dass zeitliche oder räumliche Trennungen zwischen den einzelnen Teilreaktionen erforderlich sind.

Als Alkohol-Dehydrogenasen werden vorzugsweise im Handel erhältliche oder leicht zugängliche Alkohol-Dehydrogenasen, z.B. bakterielle Enzyme von Stämmen von *Bacillus, Pseudomonas, Corynebacterium, Rhodococcus, Lactobacillus* und/oder *Thermoanaerobium,* wie z.B. jene aus Stämmen von *Rhodococcus ruber, Lactobacillus kefir* oder *Thermoanaerobium brockii* oder Enzyme aus Hefestämmen, wie *Aspergillus, Candida, Pichia* oder *Saccharomyces* eingesetzt, da diese besonders gute Ergebnisse in Bezug auf Enantiomerenüberschuss und Reaktionsgeschwindigkeit lieferten. Ausschlaggebend für die Auswahl geeigneter ADH Paare ist jedoch vor allem die Anforderung, dass die beiden ADHs entgegengesetzte Stereoselektivität und unterschiedliche Cofaktorselektivität aufweisen müssen. Die Cofaktoren ergeben sich entsprechend aus der jeweiligen Wahl der ADHs, sind in aller Regel NAD und NADP und werden vorzugsweise lediglich in katalytischen Mengen eingesetzt.

Figur 3 illustriert die Reaktionsabläufe im Verfahren der Erfindung, wobei HTS für "Hydrid-Transfer-System" steht, worunter die vom mit "Aux" bezeichneten Zusatzenzym katalysierten Nebenreaktionen zur "Regenerierung" der Cofaktoren, d.h. Überführung der oxidierten und reduzierten Formen ineinander, zu verstehen ist. $k_1$ bis $k_4$ stehen für formale Geschwindigkeitskonstanten der Reaktionen erster Ordnung des Hydridtransfersystems.

Wie in Figur 3 A skizziert, werden die Oxidations- und die Reduktions-Reaktion der sekundären Alkohol-Enantiomere von den beiden entgegengesetzt stereoseletiven ADHs (im Schema nicht dargestellt) katalysiert. Wenn die Reaktion vom (S)- zum (R)-Enantiomer abläuft, wird bei der Oxidation des (S)-Isomers von der (S)-selektiven ADH, die eine Cofaktor-Präferenz für NAD aufweist, ein Hydridion vom Alkohol abgespalten und auf die oxidierte Form des Cofaktors, $NAD^+$, übertragen, der dadurch in die reduzierte Form, NADH übergeht. Im Wesentlichen gleichzeitig übernimmt das Zusatzenzym Aux dieses Hydridion von NADH (was "Aux-H" ergibt) und überträgt es anschließend auf den zweiten Cofaktor in der oxidierten Form, $NADP^+$, wodurch dessen reduzierte Form NADPH bereitgestellt wird. Von dieser wiederum wird das Hydrid durch die zweite, (R)-selektive ADH mit NADP-Präferenz auf das Keton-Zwischenprodukt P übertragen, wodurch dieses zum (R)-Enantiomer reduziert wird. In umgekehrter Richtung, d.h. bei der Umwandlung des (R)-Isomers in die (S)-Form, erfolgen die Gegenreaktionen natürlich analog.

Wird von einem im Gleichgewicht befindlichen Enzym/Cofaktor-System ausgegangen, durchläuft ein und dasselbe Hydridion die oben erläuterten Reaktionen und landet schließlich wieder auf einem - nunmehr stereoinvertierten

- Alkoholmolekül.

Die Übertragung des Hydridions durch das Zusatzenzym von einem Cofaktor auf den anderen erfolgt in der oben beschriebenen, einfachen Form, wenn es sich beim Zusatzenzym um eine Nucleotid-Transhydrogenase handelt. In diesem Fall steht Aux-H in Figur 3 B für einen Komplex des Enzyms mit dem Hydridion. Da die getesteten Nucleotid-Transhydrogenasen jedoch keine zufrieden stellenden Ergebnisse geliefert hatten, haben die Erfinder bei ihrer Suche nach Alternativen herausgefunden, dass anstelle der das Hydrid direkt übertragenden Nucleotid-Transhydrogenase auch ein weiteres Dehydrogenase/Substrat-System deren Rolle übernehmen kann. In diesem Fall steht Aux-H für die reduzierte Form des dem Zusatzenzym entsprechenden Substrats.

Als derartige Zusatzenzyme kommen prinzipiell alle Cofaktorabhängigen Oxidoreduktasen in Frage, die die Oxidations- und Reduktionsreaktionen des zu deracemisierenden sekundären Alkohols nicht stören. Dehydrogenasen, bevorzugt Glucose-Dehydrogenase (GDH), Glucose-6-phosphat-Dehydrogenase (G6PDH) und Formiat-Dehydrogenase (FDH), haben sehr gute Resultate ergeben und sind daher bevorzugte Zusatzenzyme.

In den ersten beiden Fällen wird durch den Hydridtransfer auf das Substrat Gluconsäure oder Gluconolacton bzw. deren -6-phosphat zu Glucose bzw. -6-phosphat reduziert (was "Aux-H" ergibt) und sofort wieder oxidiert. Ähnliches erfolgt im dritten Fall mit $CO_2$, das mit Formiat als Aux-H im Gleichgewicht steht. Zwar liegt das Reaktionsgleichgewicht der Oxidation von Formiat zu $CO_2$ weit auf der Kohlendioxid-Seite, die prinzipielle Reversibilität der Reaktion wurde aber bestätigt. Da im erfindungsgemäßen Verfahren kein (bzw. kaum) Zusatzsubstrat verbraucht wird und daher nur geringe Mengen erforderlich sind, ist das System Formiat-Dehydrogenase/Formiat/$CO_2$ für die vorliegenden Zwecke durchaus geeignet, wie die späteren Beispiele zeigen werden.

Wie oben erwähnt; kommt es im erfindungsgemäßen Verfahren zu keinem stöchiometrischen Verbrauch der Reagenzien, sobald der Gleichgewichtszustand erreicht wurde. Da dieser von den speziellen Enzym/Substrat-Kombinationen und deren Selektivitäten abhängt, ist eine Vorhersage oder Vorabeinstellung unmöglich. Somit stellt sich in der Praxis dieses Gleichgewicht am Beginn des Deracemisierungsverfahrens ein. In dieser, üblicherweise wenige Minuten dauernden Phase wird eine geringe Menge an Zusatzsubstrat, d.h. Glucose oder Gluconsäure, Formiat oder $CO_2$, tatsächlich verbraucht.

In welche Richtung die Isomerisierung des sekundären Alkohols abläuft, hängt zwar primär von Stereo- und Cofaktorselektivität der beiden ADHs, in weiterer Folge aber auch von der unterschiedlichen Selektivität des Zusatzenzyms für die beiden Cofaktoren ab. Für das im obigen Schema gezeigte Beispiel, das von einer (S)-selektiven ADH mit NAD-Präferenz und einer (R)-selektiven ADH mit NADP-Präferenz ausgeht, kann die Richtung der Deracemisierung tatsächlich durch die Cofaktorselektivität des Zusatzenzyms vorgegeben werden, weswegen dieses durchaus auch als "Steuerenzym" bezeichnet werden kann. Die Cofaktorselektivität im Oxidations- bzw. Reduktionsmodus des Zusatzenzyms führen entweder dazu, dass NADH und $NADP^+$ in $NAD^+$ und NADPH (in Figur 3 B: $k_1+k_3 > k_2+k_4$) umgewandelt werden, oder dazu, dass $NAD^+$ und NADPH in NADH und $NADP^+$ (in Figur 3 B: $k_1+k_3 < k_2+k_4$) umgewandelt werden, was im ersteren Fall zur Bildung des (R)-Enantiomers und im anderen zur Bildung des (S)-Enantiomers führt. Wird das Steuerenzym oder sein Substrat weggelassen oder weist das Steuerenzym keine Selektivität für einen der beiden Cofaktoren auf (was freilich äußerst unwahrscheinlich ist), ist nicht nur keinerlei Deracemisierung, sondern - ausgehend von optisch reinen Alkoholen - sogar die umgekehrte Reaktion, d.h. Racemisierung zu beobachten, wie dies auch aus Fig. 4 hervorgeht: Die Fig. 4 A und 4 B zeigen die Reaktionsabläufe mit 2-Octanol als sekundärem Alkohol für jeweils eine Formiat-Dehydrogenase, die entgegengesetzte Cofaktorselektivität aufweisen, und Fig. 4 C zeigt jenen für ein System ohne FDH.

Die Richtung der Deracemisierung kann aber auch durch Auswahl von ADH-Paaren mit umgekehrt entgegengesetzter Stereoselektivität bzw. Cofaktorselektivität ins Gegenteil verkehrt werden. Werden beispielsweise im obigen Schema - bei gleichem Zusatzenzym - eine (S)-selektive ADH mit NADP-Präferenz und eine (R)-selektive ADH mit NAD-Präferenz eingesetzt, wird aus dem Racemat selektiv das optisch reine (S)-Enantionmer anstelle des (R)-Enantiomers gebildet.

Das erfindungsgemäße Verfahren wird üblicherweise in einem Lösungsmittel ausgewählt aus der Gruppe umfassend Wasser, ein- oder mehrphasigen Gemische aus Wasser und einem oder mehreren organischen Lösungsmittel und ionische Flüssigkeiten durchgeführt, wobei aus Kosten- und Stabilitätsgründen vorzugsweise ein herkömmliches wässriges Puffersystem zum Einsatz kommt.

Unter wässrigem Puffersystem ist ein wässriges Lösungsmittel zu verstehen, welches Substanzen, wie z.B. Salze, enthält, die das Lösungsmittel unempfindlich gegenüber pH-Wert Änderungen macht. Bekannte wässrige Puffersystem sind beispielsweise das Kohlensäure/Bicarbonat-System, der Kohlensäure-Silicat-Puffer, der Essigsäure/Acetat-Puffer, der Phosphatpuffer, der Veronal/Acetat-Puffer nach Michaelis, der Ammoniakpuffer, HEPES (4-(2-Hydroxyethyl)-1-piperazinethanesulfonsäure) und MES (2-(N-Morpholino)ethansulfonsäure).

Kurze Beschreibung der Figuren:

Es zeigt die Figur 1: Prinzip der enzymkatalysierte Racemisierung mittels zweier spezifischer ADHs unter über das prochirale Keton

Es zeigt die Figur 2: Racematspaltung mit definiertem Stammhintergrund und anschließender Cofaktorregenerierung

Es zeigt die Figur 3: A: Prinzip der erfinderischen, enzymkatalysierten Deracemisierung mittels zweier spezifischer ADHs und Hilfsenzym (Hydrid-Transfer-System: HTS) zur Cofaktorregenerierung, B: Prinzip des Hydrid-Transfer-Systems

Es zeigt die Figur 4: Verschiebung des racemischen Gemisches von 1-Phenylethanol: A und B: mit Formiatdehydrogenase (FDH), C: ohne Formiatdehydrogenase (FDH)

Es zeigt die Figur 5: Verschiebung des racemischen Gemisches mittels NAD- oder NADP-spezifischer Formiatdehydrogenase (FDH) Es zeigt die Figur 6: Einfluss der Variation verschiedener Reaktionsparamter auf das Reaktionsgleichgewicht

Es zeigt die Figur 7: Einfluss verschiedener Konzentrationen des Zusatzsubstrats Glucose auf die Einstellung des racemischen Gleichgewichts, A: zeitlicher Verlauf für verschiedene Glucosekonzentrationen, B:Korrelation ee [%] zu den jeweiligen Glucosekonzentrationen nach 6 Stunden Reaktionszeit

**Beispiele:**

Die Erfindung wird nun anhand von repräsentativen Ausführungsbeispielen näher beschrieben.

*Materialien, Bezugsquellen und Verfahren*

*Enzyme*

ADH-A: Alkohol-Dehydrogenase von *Rhodococcus ruber* (im Handel erhältlich von BioCatalytics Inc., nunmehr Codexis, Pasadena, USA).

LK-ADH: Alkohol-Dehydrogenase von *Lactobacillus kefir* (im Handel erhältlich von Sigma-Aldrich, Wien, #05643, 0,4 IE/mg).

RE-ADH: Alkohol-Dehydrogenase von *Rhodococcus erythropolis* (im Handel erhältlich von Sigma-Aldrich, #68482, 20 IE/ml).

LB-ADH: Alkohol-Dehydrogenase 002 (im Handel erhältlich von Jülich Chiral Solutions, nunmehr Codexis, #05.11).

ADH-T: Alkohol-Dehydrogenase 005 (im Handel erhältlich von Jülich Chiral Solutions, nunmehr Codexis, #26.10).

ADH-PR2: Alkohol-Dehydrogenase 007 (im Handel erhältlich von Jülich Chiral Solutions, nunmehr Codexis, #42.10).

TB-ADH: Alkohol-Dehydrogenase von *Thermoanaerobium brockii* (im Handel erhältlich von Sigma-Aldrich, #A9287, 30-90 IE/mg).

G6PDH: Glucose-6-phosphat-Dehydrogenase von Bäckerhefe (im Handel erhältlich von Sigma-Aldrich, #49271, 240 IE/mg).

GLY-DH: Glycerin-Dehydrogenase von *Geotrichum candidum* (im Handel erhältlich von Sigma-Aldrich, #49860, 30 IE/mg).

LDH-SC: D-Lactat-Dehydrogenase von *Staphylococci* (im Handel erhältlich von Sigma-Aldrich, #17847, 120 IE/mg).

LDH-LS: D-Lactat-Dehydrogenase von *Lactobacillus sp.* (im Handel erhältlich von Sigma-Aldrich, #59023, 400 IE/mg).

LDH-RM: L-Lactat-Dehydrogenase aus Kaninchenmuskel (im Handel erhältlich von Sigma-Aldrich, #61311, 500IE/mg).

FDH1: NADP-spezifische Formiat-Dehydrogenase 001 (im Handel erhältlich von Jülich Chiral Solutions, nunmehr Codexis, Pasadena, USA, #25.10, 47 IE/ml).

FDH2: NAD-spezifische Formiat-Dehydrogenase 002 (im Handel erhältlich von Jülich Chiral Solutions, nunmehr Codexis, #24.11, 200 IE/ml).

FDH3: NAD-spezifische Formiat-Dehydrogenase 001 (im Handel erhältlich von Jülich Chiral Solutions, nunmehr Codexis, #09.11, 200 IE/ml).

FDH4: Formiat-Dehydrogenase von Hefe (im Handel erhältlich von der Boehringer Mannheim GmbH, #204226, 0,5 IE/mg).

FDH5: Formiat-Dehydrogenase von *Candida boidinii* (Geschenk von Martina Pohl, Universität Düsseldorf, Deutschland).

GDH-BM: D-Glucose-Dehydrogenase 001 (im Handel erhältlich von Jülich Chiral Solutions, nunmehr Codexis, #22.10, 30 IE/mg).

GDH-BS: D-Glucose-Dehydrogenase 002 (im Handel erhältlich von Jülich Chiral Solutions, nunmehr Codexis,

#29.10, 500 IE/ml).

Eigenschaften der Enzyme

Tabelle 1: Alkohol-Dehydrogenasen

| Nr. | Enzym | Cofaktor-Selektivität | Stereoselektivität |
|---|---|---|---|
| 1 | ADH-A | NAD | (S) |
| 2 | LK-ADH | NADP | (R) |
| 3 | RE-ADH | NAD | (S) |
| 4 | LB-ADH | NADP | (R) |
| 5 | ADH-T | NADP | (S) |
| 6 | ADH-PR2 | NAD | (R) |
| 7 | TB-ADH | NADP | (S) |

Tabelle 2: Zusatzenzyme

| Nr. | Enzym | Cofaktor-Selektivität[a] | Reduziertes Zusatzsubstrat |
|---|---|---|---|
| 1 | FDH1 | NADP | Formiat |
| 2 | FDH2 | NAD | Formiat |
| 3 | FDH3 | NAD | Formiat |
| 4 | FDH4 | unbekannt [b] | Formiat |
| 5 | FDH5 | unbekannt [b] | Formiat |
| 6 | GDH-BS | NAD und NADP | α-D-Glucose |
| 7 | GDH-BM | NAD und NADP | α-D-Glucose |
| 8 | G6PDH | NADP | α-D-Glucose-6-phosphat |
| 9 | GlyDH | NAD | Glycerin |
| 10 | LDH-SC | unbekannt [b] | Lactat |
| 11 | LDH-LS | unbekannt [b] | Lactat |
| 12 | LDH-RM | unbekannt [b] | Lactat |

a: Daten entweder aus der Literatur oder vom Hersteller. b: Keine Daten gefunden.

*Chemikalien*

rac-2-Octanol (#O4504, MG 130,23 g/mol), (R)-2-Octanol (#74864, MG 130,23 g/mol), (S)-2-Octanol (#74863, MG 130,23 g/mol), 2-Octanon (#53220, MG 128,21 g/mol), Ammoniumformiate (#09739, 63,06 g/mol), Natriumformiat (#3996-15-4, 69,02 g/mol) und Ameisensäure als Kaliumsalz (#57444-81-2, MG 85,13 g/mol) wurden von Sigma-Aldrich, Wien, bezogen.

Chemikalien für Extraktion und Aufarbeitung:

Ethylacetat (#441977) zur Extraktion wurde von der Brenntag CEE GmbH, Ort, bezogen und frisch destilliert eingesetzt. DMAP (#29224, MG 122,17 g/mol) und Essigsäureanhydrid (#45830, MG 102,09 g/mol) zur Acetylierung wurden von Sigma-Aldrich, Wien, bezogen.

*Allgemeine Vorgangsweise*

Modellverfahren zur Verschiebung der optischen Zusammensetzung: Die Aktivität der handelsüblichen Enzyme ist allgemein in internationalen Einheiten (IE) angegeben. Diese Einheiten geben jedoch allesamt die Aktivität des jeweiligen Enzyms für ein anderes Substrat, als hierin eingesetzt wurde, an. Daher wurde die Aktivität der verwendeten Enzyme bei der Reduktion von 2-Octanon mit einem geeigneten "Regenerations"-System bestimmt (im Allgemeinen

eine FDH mit Ammoniumformiat, 5 Äqu.). Für alle Versuche wurde etwa 1 $IE_{2\text{-Octanol}}$ der ADHs eingesetzt.

ADH-A, LK-ADH, NADP-spezifische FDH (2 IE), Ammoniumformiat (3 Äqu. der Substratkonzentration), $NAD^+$ und $NADP^+$ (3 Mol-% des Substrats) wurden in TRIS-HCl (50 mM, pH 7,5, Gesamtvolumen 0,5 ml) suspendiert. Die Reaktion wurde durch Zusatz von racemischem 2-Octanol gestartet (0,5 μl, 8 mmol/ml, ee < 3%). Nach 3 h Schütteln (130 U/min) bei 30 ˚C wurde das Gemisch mit EtOAc (500 μl) extrahiert und zentrifugiert, um Phasentrennung zu bewirken.

*Analysenverfahren*

Chirale GC-FID-Analyse:

Die Alkohole wurden durch Zusatz von Essigsäureanhydrid (100 ml) und DMAP (0,5 mg) innerhalb von 2 h bei 30 ˚C acetyliert. Nach der Aufarbeitung wurden die Produkte mittels GC-FID und GC-MSD mit einer chiralen staionären Phase analysiert.

Chirale GC-FID-Analysen erfolgten an einem Varian-3900-Gaschromatographen mit einem FID-Detektor unter Verwendung einer Chrompack-Chirasil-DEX-CB-Säule (Varian, 25 m x 0,32 mm x 0,25 mm, 1,0 bar $H_2$), Detektortemperatur 250 ˚C, Teilungsverhältnis 90:1.

Chirale GC-MSD-Analyse:

Chirale GC-MSD-Analysen erfolgten an einem Agilent-7890A-GC-System mit einem massenselektiven Agilent-5975C-Detektor und

einem FID unter Verwendung einer Chrompack-Chirasil-DEX-CB-Säule (Varian, 25 m x 0,32 mm x 0,25 mm, 1,0 bar $H_2$),
Detektortemperatur 250 ˚C, Teilungsverhältnis 90:1.

Chirale HPLC-Analyse:

HPLC-Analysen erfolgten an einem Shimadzu-HPLC-System mit DGU-20A5-Entgaser, LC-20AD-Flüssigchromatographen, SIL-20AC-Autosampler, CBM-20A-Kommunikationsbusmodul, SPD-M20A-Diodenarray-Detektor und CTO-20AC-Säulenofen unter Verwendung einer Chiralpak-AD-Säule (Daicel, 0,46 x 25 cm) mit n-Heptan/Isopropanol = 90:10, 0,5 ml/min, 18 ˚C.

*Beispiele 1 bis 13, Vergleichsbeispiele 1 bis 4*

Deracemisierungen wurden unter Verwendung verschiedener ADH/Zusatzenzym-Kombinationen mit 2-Octanol als sekundärem Alkohol unter folgenden Bedingungen durchgeführt: Substratkonzentration 8 mmol/l, Reaktionszeit 3-12 h, 30 ˚C in TRIS-HCl (pH 7,5, 50 mM) oder Phosphatbuffer (pH 7,5, 50 mM), Schütteln mit 130 U/min. Von den ADHs jeweils etwa 1 IE (für 2-Octanol als Substrat); $NAD^+$ and $NADP^+$ in katalytischen Mengen (ca. 3 Mol-%). Zusatzenzyme: 2 IE (für ihr natürliches Substrat, wie vom Hersteller angegeben). Zusatzsubstrate (Formiat, Glucose, Glucose-6-phosphat, Lactat und Glycerin): 16 mmol/l. Die Zusammensetzungen und Ergebnisse sind in nachfolgender Tabelle 3 zusammengefasst.

Tabelle 3: Deracemisierung von *rac*-2-Octanol

| **Beispiel / Vergl.beispiel** | | **Enzyme** | | **Produkt** | |
|---|---|---|---|---|---|
| | | **Alkohol-Dehydrogenasen, ADHs** | **Zusatzenzym** | **ee [%]** | **Alkohol [%]** |
| ***B1*** | | ADH-A + LK-ADH | NADP-spezifische FDH 001 | >99 (R) | >99% |
| **B2** | | | NAD-spezifische FDH 002 | >99 (S) | >99% |
| **B3** | | | NAD-spezifische FDH 001 | >99 (S) | >99% |
| **B4** | | | FDH4 | 61 (S) | >99% |
| **B5** | | | GDH-BS | >99 (R) | >99% |

(fortgesetzt)

| Beispiel / Vergl.beispiel | | Enzyme | | Produkt | |
|---|---|---|---|---|---|
| | | Alkohol-Dehydrogenasen, ADHs | Zusatzenzym | ee [%] | Alkohol [%] |
| B6 | | | GDH-BM | 41 (R) | >99% |
| B7 | | | G6P-DH | >99 (R) | >99% |
| V1 | | | LDH-SC | rac | >99% |
| V2 | | | LDH-LS | rac | 96% |
| V3 | | | LDH-RM | rac | >99% |
| V4 | | | GlyDH | rac | 97% |
| B8 | | ADH-T + ADH-PR2 | NADP-spezifische FDH 001 | >99 (S) | >99% |
| B9 | | | NAD-spezifische FDH 002 | >99 (R) | >99% |
| B10 | | RE-ADH + LB-ADH | NADP-spezifische FDH 001 | 94 (R) | >99% |
| B11 | | | NAD-spezifische FDH 002 | 34 (S) | >99% |
| B12 | | *Thermoanaerobium* brokii-ADH + ADH-PR2 | NADP-spezifische FDH 001 | 89 (S) | >99% |
| B13 | | | NAD-spezifische FDH 002 | 96 (R) | >99% |

Es ist zu erkennen, dass die getesteten Enzym-Kombinationen der Beispiele 1 bis 13 der vorliegenden Erfindung aus 2-Octanol-Racematen in überwiegend gutem, zum Teil praktisch quantitativem Enantiomerenüberschuss und in fast durchwegs quantitativer Ausbeute eines der Enantiomere lieferten. Bei Verwendung desselben ADH-Paars konnte durch Umkehr der Cofaktorspezifität des Zusatzenzyms die Richtung der Deracemisierung gesteuert werden: vgl. die Beispiele 1/2+3, 8/9, 10/11, 12/13. Das Ergebnis der Beispiele 8 und 9 ist auch in Fig. 5 grafisch dargestellt.

Bei Verwendung von Lactat- oder Glycerin-Dehydrogenase in den Vergleichsbeispielen 1 bis 4 erfolgte hingegen keinerlei Deracemisierung.

*Beispiele 14 bis 22*

In diesen Beispielen wurden unter Verwendung des Enzymsystems aus Beispiel 1 verschiedene Reaktionsparameter variiert, um deren Auswirkung auf den Reaktionsverlauf zu untersuchen. Die Ergebnisse der Beispiele 14 bis 21 sind in den Fig. 6 A-H grafisch dargestellt, jene von Beispiel 22 in den Fig. 7 A-B.

*Beispiel 14*

Hier wurde die Reaktionszeit zwischen 1 und 6 h variiert und herausgefunden, dass nach 3 h bereits quantitativer Umsatz erreicht war. Die weiteren Beispiele dieser Gruppe wurden daher 3 h lang durchgeführt (Fig. 6 A).

*Beispiel 15*

Die Alkoholkonzentration wurde zwischen 1 und 243 mmol/l variiert, wobei 2 bis 8 mmol/l die besten Ergebnisse bei der gegebenen Reaktionszeit von 6 h lieferten. Bei höheren Konzentrationen ist entweder eine längere Reaktionszeit oder eine größere Menge an Enzym vonnöten, um vollständigen Umsatz zu erzielen (Fig. 6 B).

*Beispiel 16*

Die Gesamtmenge der beiden ADHs wurde zwischen 0,1 und 3,4 IE variiert, wobei sich 1 IE als optimale Aktivitätsmenge herausstellte (Fig. 6 C).

*Beispiel 17*

Das Aktivitätsverhältnis (in IE) der beiden ADHs zueinander wurde zwischen 0,01 und 13,5 variiert und herausgefunden, dass ein Verhältnis zwischen etwa 0,2 und etwa 0,7 am effektivsten war, wobei allerdings ein Wert für ein 1:1-Verhältnis fehlte (Fig. 6 D).

*Beispiele 18 und 19*

Die Aktivität jeweils einer der beiden ADHs wurde zwischen 0,1 und 7,6 bzw. 3,4 IE variiert, wobei die Aktivität der zweiten ADH 1 IE betrug, und herausgefunden, dass 1 IE auch die optimale Aktivitätsmenge für das zweite Enzym darstellt und daher 1:1 das optimale Aktivitätsverhältnis der beiden ADHs ist (Fig. 6 E, 6 F).

*Beispiel 20*

Die Menge an FDH wurde zwischen 0,3 und 64,0 IE variiert, wobei sich herausstellte, dass ab einer Menge von 2 IE bereits quantitativer Umsatz erzielt wird (Fig. 6 G).

*Beispiel 21*

Die gemeinsame Konzentration der Cofaktoren NAD und NADP wurde zwischen 0 und 96 Mol-% variiert, wobei eine Konzentration von etwa 2 bis 3 Mol-% am wirksamsten war (Fig. 6 H).

*Beispiel 22*

Beispiel 5 wurde wiederholt, wobei die Konzentration des Zusatzsubstrats, d.h. Glucose, zwischen 0,1 und 3 Äquivalenten der Alkoholkonzentration über eine Reaktionszeit zwischen 0,5 und 12 h variiert wurde, wie in Fig. 7 A gezeigt. Der Enantiomerenüberschuss ee nach 6 h bei Variation der Glucose-Äquivalente zwischen 0,1 und 1 wird in Fig. 7 B gezeigt. Dabei wurden >99% ee bereits ab 0,3 Äquivalenten erzielt, was zeigt, dass auch ein deutlich unterstöchiometrischer Anteil an Zusatzsubstrat ausreicht.

*Beispiele 23 bis 32*

In diesen Beispielen wurde unter Verwendung des Enzymsystems aus Beispiel 1 versucht, Racemate von 10 anderen sekundären Alkoholen zu deracemisieren. Die Auswahl der Alkohole wurde unter Berücksichtigung der in der Fachliteratur beschriebenen Substratspektren für die beiden involvierten ADHs getroffen. Prinzipiell sollten auf diese Weise alle Substrate nach dem erfindungsgemäßen Verfahren deracemisierbar sein, die im Substratspektrum beider ADHs enthalten sind. Die Strukturen der in diesen Beispielen eingesetzten sekundären Alkohole sind in der folgenden Tabelle 4 angeführt.

Tabelle 4: Substrate der Beispiele 1 und 23 bis 32

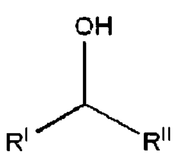

1

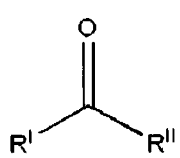

2

| Beispiel | $R^I$ | $R^{II}$ | Name |
|---|---|---|---|
| 1 | $CH_3$ | $C_6H_{13}$ | 2-Octanol |
| 23 | $CH_3$ | $C_7H_{15}$ | 2-Nonanol |
| 24 | $CH_3$ | $C_8H_{17}$ | 2-Decanol |
| 25 | $CH_3$ | 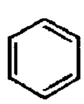 | 1-Phenyl-1-ethanol |

(fortgesetzt)

| Beispiel | $R^I$ | $R^{II}$ | Name |
|---|---|---|---|
| 26 | $CH_3$ | 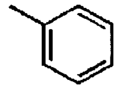 | 1-Phenyl-2-propanol |
| 27 | $CH_3$ | 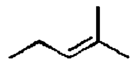 | Sulcatol |
| 28 | $C_2H_5$ | $C_SH_{11}$ | 3-Octanol |
| 29 | $C_2H_5$ | $C_6H_{13}$ | 3-Nonanol |
| 30 | $C_2H_5$ | $C_7H_{15}$ | 3-Decanol |
| 31 | 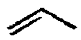 | $C_6H_{13}$ | 1-Octen-3-ol |
| 32 | -"- | $C_5H_{11}$ | 1-Hepten-3-ol |

Die Ergebnisse der Racemisierungen sind in nachfolgender Tabelle 5 zusammengefasst.

Tabelle 5: Ergebnisse der Deracemisierung von Racematen sekundärer Alkohole

| Substrate[a)] | Beispiel | Zeit [h] | Alkohol [%] | Enantiomer | *ee* [%] |
|---|---|---|---|---|---|
| *rac*-2-Octanol | 1 | 3 | 99 | (*R*) | >99 |
| *rac*-2-Nonanol | 23 | 3 | 97 | (*R*) | >99 |
| *rac*-2-Decanol | 24 | 3 | 99 | (*R*) | >99 |
| *rac*-1-Phenyl-ethanol | 25 | 2 | | (*R*) | |
| | | | >99 | | >99 |
| *rac*-1-Phenyl-2-propanol | 26 | 2 | | (*R*) | |
| | | | 98 | | 80,5 |
| *rac*-Sulcatol | 27 | 3 | 95 | (*R*) | >99 |
| *rac*-3-Octanol | 28 | 4 | 98 | (*R*) | >99 |
| *rac*-3-Nonanol | 29 | 4 | 97 | (*R*) | >99 |
| *rac*-3-Decanol | 30 | 4 | >99 | (*R*) | 98 |
| *rac*-1-Octen-3-ol | 31 | 3 | 99 | (*S*) [b)] | 95 |
| *rac*-1-Hepten-3-ol | 32 | 3 | 93 | (*S*) [b)] | 96 |

a): ee der racemischen Substrate <3%.
b): Änderung der Cahn-Ingold-Prelog-Priorität

Aus der Tabelle geht klar hervor, dass sämtliche getesteten Racemate nach dem erfindungsgemäßen Verfahren mit ausgezeichneter Selektivität in kurzer Zeit nahezu quantitativ deracemisiert werden konnten, wobei die Gegenwart weiterer Funktionalitäten die Wirksamkeit des erfindungsgemäßen Verfahrens nicht schmälerte.

Die vorliegende Erfindung stellt somit eine wertvolle Bereicherung des Gebiets der Stereoisomerisierung dar, weswegen an der gewerblichen Anwendbarkeit der Erfindung kein Zweifel besteht.

## Patentansprüche

1. Verfahren zur enzymatischen Deracemisierung von Enantiomerengemischen sekundärer Alkohole durch eine Kombination aus Oxidations- und Reduktionsreaktionen mittels stereoselektiver Alkohol-Dehydrogenasen und derer Cofaktoren, wobei formal ein Enantiomer eines optisch aktiven sekundären Alkohols selektiv zum entsprechenden Keton oxidiert wird, das anschließend selektiv zum optischen Antipoden reduziert wird, während die reduzierte Form des Cofaktors für die Reduktionsreaktion über ein Zusatzenzym zur Verfügung gestellt wird, **dadurch gekennzeichnet, dass** zwei Alkohol-Dehydrogenasen mit entgegengesetzter Stereoselektivität und unterschiedlicher Cofaktorselektivität sowie die zwei zugehörigen, unterschiedlichen Cofaktoren für die Oxidations-

bzw. die Reduktionsreaktion eingesetzt und die oxidierten und reduzierten Cofaktoren in einer parallel ablaufenden enzymatischen Reaktion mit dem Zusatzenzym ineinander übergeführt werden, wobei die Richtung der Deracemisierung hin zu einem der beiden Enantiomere durch die Wahl der beiden Alkohol-Dehydrogenasen bzw. anhand des Selektivitätsunterschieds des Zusatzenzyms für die beiden Cofaktoren steuerbar ist.

2. Verfahren nach Anspruch 1, **dadurch gekennzeichnet, dass** als Alkohol-Dehydrogenasen bakterielle Alkohol-Dehydrogenasen eingesetzt werden.

3. Verfahren nach Anspruch 1 oder 2, **dadurch gekennzeichnet, dass** als Alkohol-Dehydrogenasen Alkohol-Dehydrogenasen aus *Bacillus, Pseudomonas, Corynebacterium, Rhodococcus, Lactobacillus* oder *Thermoanaerobium* eingesetzt werden.

4. Verfahren nach Anspruch 1, **dadurch gekennzeichnet, dass** als Alkohol-Dehydrogenasen Enzyme aus Hefestämmen eingesetzt werden.

5. Verfahren nach Anspruch 4, **dadurch gekennzeichnet, dass** als Alkohol-Dehydrogenasen Alkohol-Dehydrogenasen aus *Aspergillus, Candida, Pichia* oder *Saccharomyces* eingesetzt werden.

6. Verfahren nach einem der Ansprüche 1 bis 5, **dadurch gekennzeichnet, dass** die beiden Alkohol-Dehydrogenasen in einem Aktivitätsverhältnis von 1:1 eingesetzt werden.

7. Verfahren nach einem der vorangegangenen Ansprüche, **dadurch gekennzeichnet, dass** die beiden Alkohol-Dehydrogenasen in einer Gesamtmenge von 1 IE eingesetzt werden.

8. Verfahren nach einem der vorangegangenen Ansprüche, **dadurch gekennzeichnet, dass** der racemische sekundäre Alkohol in einer Konzentration von zumindest 2 mmol/l eingesetzt wird.

9. Verfahren nach einem der vorangegangenen Ansprüche, **dadurch gekennzeichnet, dass** als Zusatzenzym eine Glucose-Dehydrogenase, Glucose-6-phosphat-Dehydrogenase, Formiat-Dehydrogenase oder Nucleotid-Transhydrogenase eingesetzt wird.

10. Verfahren nach einem der vorangegangenen Ansprüche, **dadurch gekennzeichnet, dass** das Zusatzenzym in einer Menge von 2 IE eingesetzt wird.

11. Verfahren nach einem der vorangegangenen Ansprüche, **dadurch gekennzeichnet, dass** das Substrat des Zusatzenzyms in einer Menge von zumindest 0,3 mol pro Mol des sekundären Alkohols eingesetzt wird.

12. Verfahren nach einem der vorangegangenen Ansprüche, **dadurch gekennzeichnet, dass** die Cofaktoren in katalytischen Mengen eingesetzt werden.

13. Verfahren nach Anspruch 9, **dadurch gekennzeichnet, dass** die Cofaktoren in einer Menge von 2 bis 3 Mol-%, bezogen auf den sekundären Alkohol, eingesetzt werden.

14. Verfahren nach einem der vorangegangenen Ansprüche, **dadurch gekennzeichnet, dass** ein aus Wasser, ein- oder mehrphasigen Gemischen aus Wasser und einem oder mehreren organischen Lösungsmitteln sowie ionischen Flüssigkeiten ausgewähltes Lösungsmittel eingesetzt wird.

15. Verfahren nach Anspruch 14, **dadurch gekennzeichnet, dass** als Lösungsmittel ein wässriges Puffersystem eingesetzt wird.

## Claims

1. Process for enzymatic deracemization of enantiomer mixtures of secondary alcohols by a combination of oxidation and reduction reactions by means of stereoselective alcohol dehydrogenases and the cofactors thereof, wherein one enantiomer of an optically active secondary alcohol is in a formal sense selectively oxidized to the corresponding ketone, which is subsequently reduced selectively to the optical antipode, while the reduced form of the cofactor is provided for the reduction reaction by means of an additional enzyme,

**characterized in that** two alcohol dehydrogenases with opposite stereoselectivity and different cofactor selectivity and the two corresponding, different cofactors are used for the oxidation and reduction reactions, and the oxidized and reduced cofactors are interconverted in a parallel enzymatic reaction with the additional enzyme, the direction of the deracemization toward one of the two enantiomers being controllable by the selection of the two alcohol dehydrogenases or using the selectivity difference of the additional enzyme for the two cofactors.

**2.** Process according to Claim 1, **characterized in that** the alcohol dehydrogenases used are bacterial alcohol dehydrogenases.

**3.** Process according to Claim 1 or 2, **characterized in that** the alcohol dehydrogenases used are alcohol dehydrogenases from *Bacillus, Pseudomonas, Corynebacterium, Rhodococcus, Lactobacillus* or *Thermoanaerobium.*

**4.** Process according to Claim 1, **characterized in that** the alcohol dehydrogenases used are enzymes from yeast strains.

**5.** Process according to Claim 4, **characterized in that** the alcohol dehydrogenases used are alcohol dehydrogenases from *Aspergillus, Candida, Pichia* or *Saccharomyces.*

**6.** Process according to any one of Claims 1 to 5, **characterized in that** the two alcohol dehydrogenases are used in an activity ratio of 1:1.

**7.** Process according to any one of the preceding claims, **characterized in that** the two alcohol dehydrogenases are used in a total amount of 1 IE.

**8.** Process according to any one of the preceding claims, **characterized in that** the racemic secondary alcohol is used in a concentration of at least 2 mmol/l.

**9.** Process according to any one of the preceding claims, **characterized in that** the additional enzyme used is a glucose dehydrogenase, glucose 6-phosphate dehydrogenase, formate dehydrogenase or nucleotide transhydrogenase.

**10.** Process according to any one of the preceding claims, **characterized in that** the additional enzyme is used in an amount of 2 IE.

**11.** Process according to any one of the preceding claims, **characterized in that** the substrate of the additional enzyme is used in an amount of at least 0.3 mol per mole of secondary alcohol.

**12.** Process according to any one of the preceding claims, **characterized in that** the cofactors are used in catalytic amounts.

**13.** Process according to Claim 9, **characterized in that** the cofactors are used in an amount of 2 to 3 mol%, based on the secondary alcohol.

**14.** Process according to any one of the preceding claims, **characterized in that** a solvent selected from water, mono- or polyphasic mixtures of water and one or more organic solvents, and ionic liquids is used.

**15.** Process according to Claim 14, **characterized in that** the solvent used is an aqueous buffer system.

## Revendications

**1.** Procédé de déracémisation enzymatique de mélanges d'énantiomères d'alcools secondaires par une combinaison de réactions d'oxydation et de réduction à l'aide d'alcool-déshydrogénases stéréo-sélectives et de leurs co-facteurs, procédé dans lequel un énantiomère d'un alcool secondaire optiquement actif est d'une manière formelle sélectivement oxydé en la cétone correspondante, laquelle est ensuite sélectivement réduite en son énantiomère optique, tandis que la forme réduite du co-facteur est mise à la disposition de la réaction de réduction par l'intermédiaire d'une enzyme additionnelle, **caractérisé en ce qu'**on utilise deux alcool-déshydrogénases, ayant des stéréo-sélectivités opposées et des sélectivités différentes de co-facteurs, ainsi que les deux co-facteurs correspondants,

différents, pour la réaction d'oxydation et de réduction, et les co-facteurs oxydés et réduits sont, dans le cadre d'une réaction enzymatique se déroulant en parallèle avec l'enzyme additionnelle, convertis l'un en l'autre, la direction de la déracémisation pouvant être pilotée vers l'un des deux énantiomères grâce au choix des deux alcool-déshydrogénases, ou à l'aide de la différence de sélectivité de l'enzyme additionnelle pour les deux co-facteurs.

**2.** Procédé selon la revendication 1, **caractérisé en ce qu'**on utilise en tant qu'alcool-déshydrogénases des alcool-déshydrogénases bactériennes.

**3.** Procédé selon la revendication 1 ou 2, **caractérisé en ce qu'**on utilise en tant qu'alcool-déshydrogénases des alcool-déshydrogénases de *Bacillus, Pseudomonas, Corynebacterium, Rhodococcus, Lactobacillus ou Thermoanaerobium.*

**4.** Procédé selon la revendication 1, **caractérisé en ce qu'**on utilise en tant qu'alcool-déshydrogénases des enzymes provenant de souches de levures.

**5.** Procédé selon la revendication 4, **caractérisé en ce qu'**on utilise en tant qu'alcool-déshydrogénases des alcool-déshydrogénases d'*Aspergillus,* de *Candida,* de *Pichia* ou de *Saccharomyces.*

**6.** Procédé selon l'une des revendications 1 à 5, **caractérisé en ce que** les deux alcool-déshydrogénases sont utilisées selon un rapport entre activités de 1:1.

**7.** Procédé selon l'une des revendications précédentes, **caractérisé en ce que** les deux alcool-déshydrogénases sont utilisées en une quantité totale de 1 UI.

**8.** Procédé selon l'une des revendications précédentes, **caractérisé en ce que** l'alcool secondaire racémique est utilisé à une concentration d'au moins 2 mmol/l.

**9.** Procédé selon l'une des revendications précédentes, **caractérisé en ce qu'**on utilise en tant qu'enzyme additionnelle une glucose-déshydrogénase, une glucose-6-phosphate-déshydrogénase, une formiate-déshydrogénase ou une nucléotide-transhydrogénase.

**10.** Procédé selon l'une des revendications précédentes, **caractérisé en ce que** l'enzyme additionnelle est utilisée en une quantité de 2 UI.

**11.** Procédé selon l'une des revendications précédentes, **caractérisé en ce que** le substrat de l'enzyme additionnelle est utilisé en une quantité d'au moins 0,3 mol par mole de l'alcool secondaire.

**12.** Procédé selon l'une des revendications précédentes, **caractérisé en ce que** les co-facteurs sont utilisés en des quantités catalytiques.

**13.** Procédé selon la revendication 9, **caractérisé en ce que** les co-facteurs sont utilisés en une quantité de 2 à 3 % en moles par rapport à l'alcool secondaire.

**14.** Procédé selon l'une des revendications précédentes, **caractérisé en ce qu'**on utilise un solvant choisi parmi l'eau, les mélanges mono- ou polyphasiques d'eau et d'un ou plusieurs solvants organiques, ainsi que les liquides ioniques.

**15.** Procédé selon la revendication 14, **caractérisé en ce qu'**on utilise en tant que solvant un système tampon aqueux.

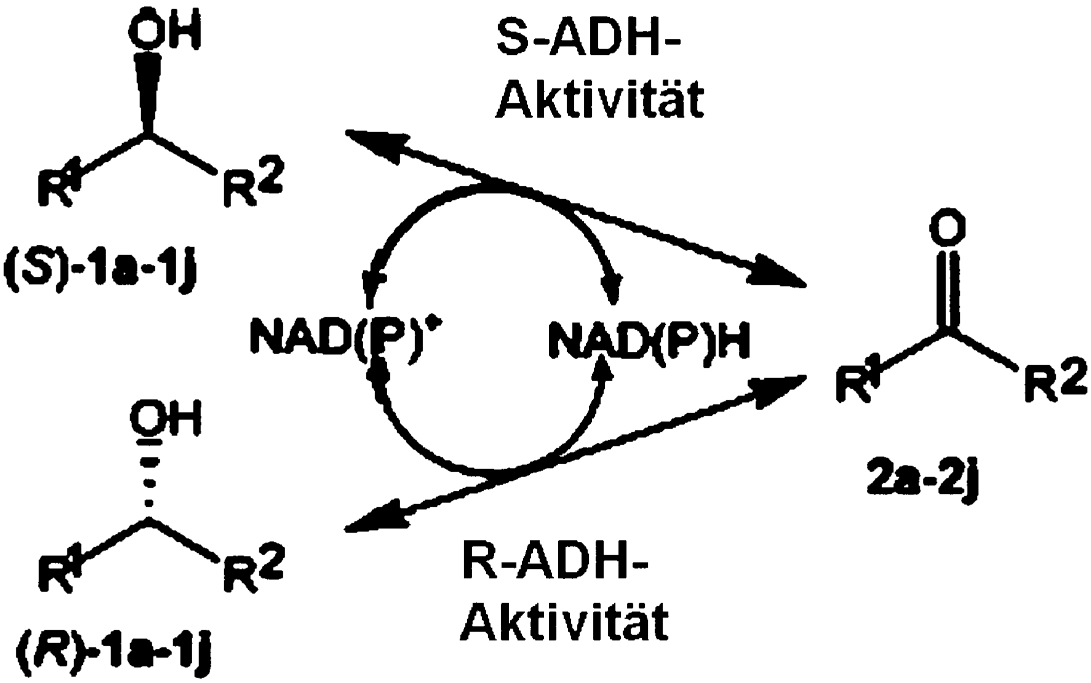


(*S*)-1a-1j

(*R*)-1a-1j

2a-2j

Figur 1

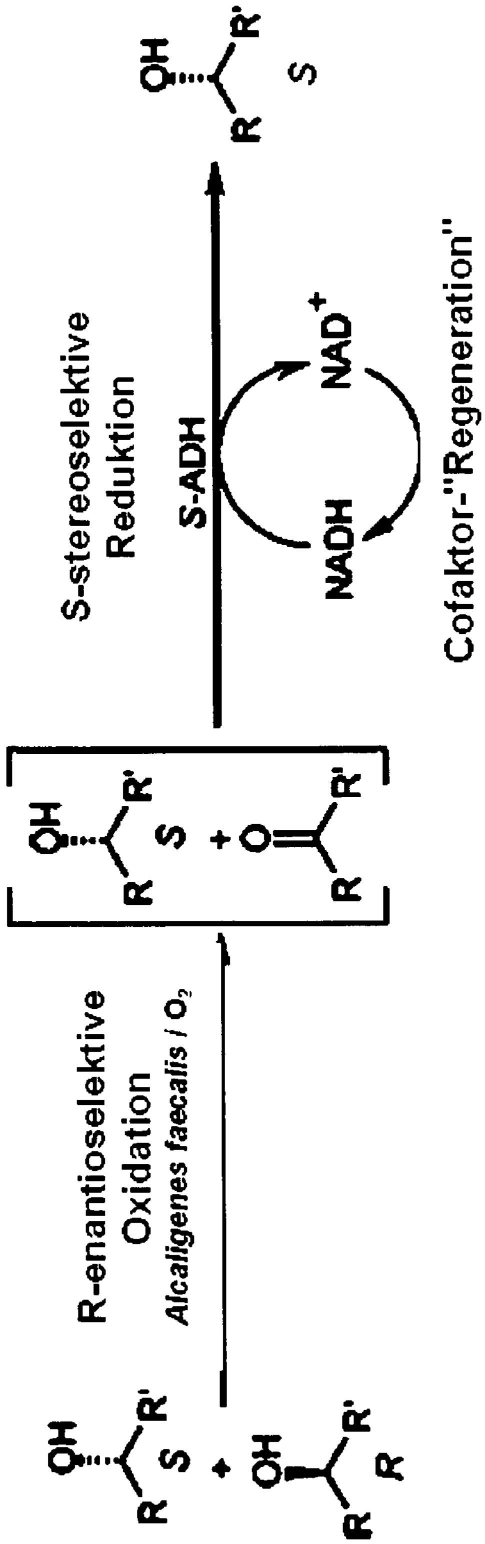
R-enantioselektive
Oxidation
*Alcaligenes faecalis* / $O_2$
S-stereoselektive
Reduktion
S-ADH
NADH
$NAD^+$
Cofaktor-"Regeneration"

Figur 2

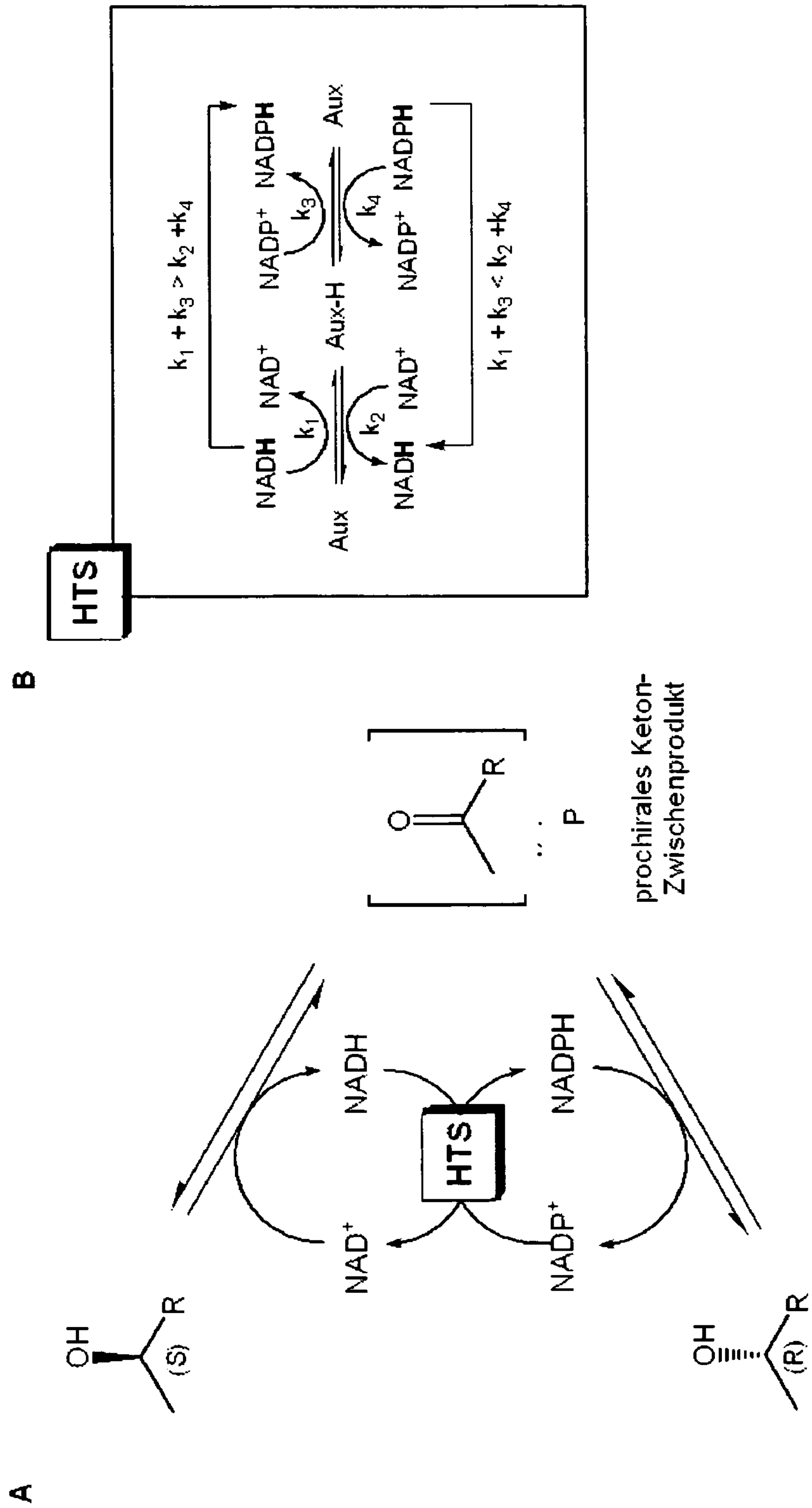
A
B
HTS
NADH
NAD+
NADPH
NADP+
Aux
Aux-H
k1 + k3 > k2 + k4
k1 + k3 < k2 + k4
prochirales Keton-
Zwischenprodukt

Figur 3

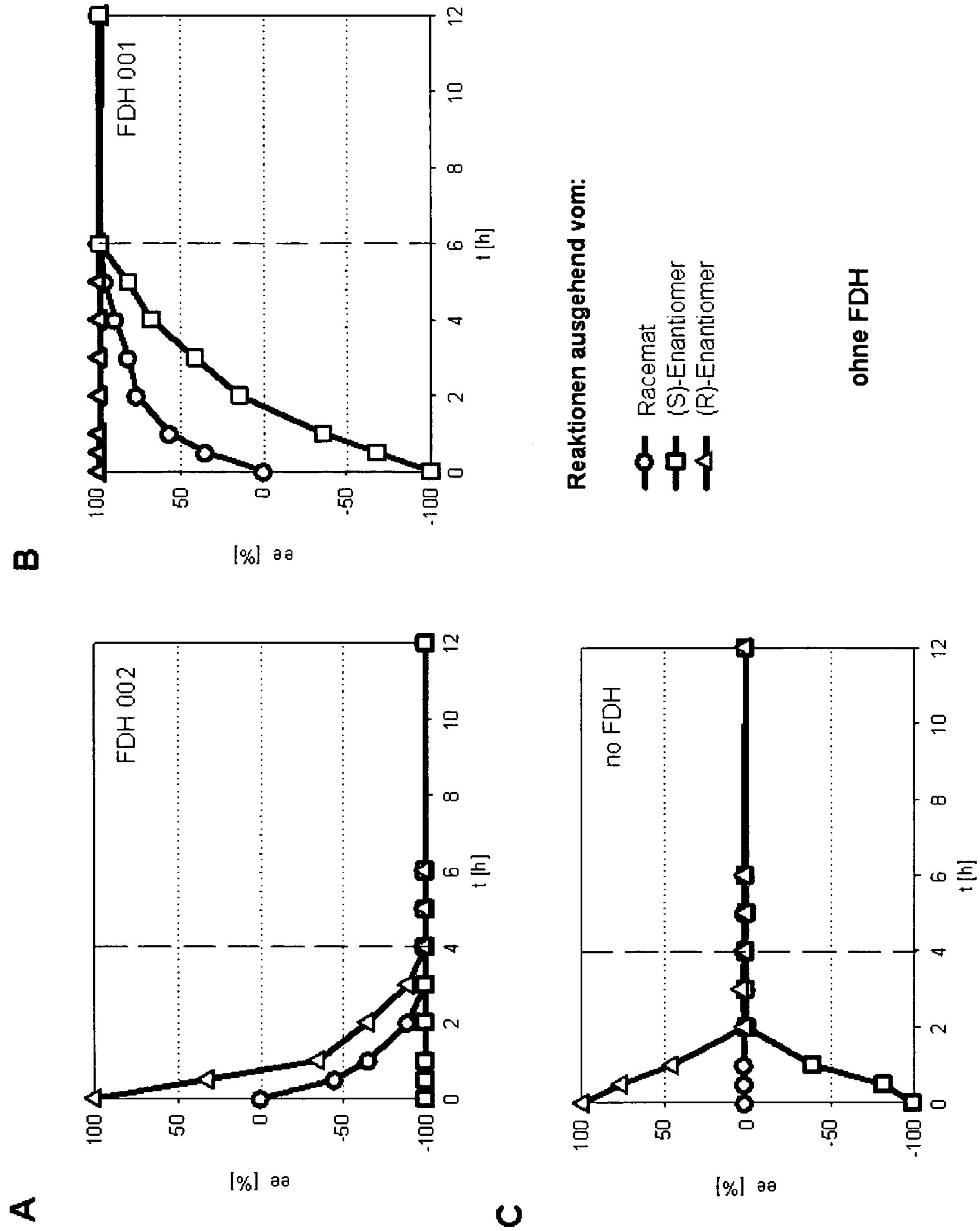
A
FDH 002
B
FDH 001
C
no FDH
ee [%]
t [h]
Reaktionen ausgehend vom:
Racemat
(S)-Enantiomer
(R)-Enantiomer
ohne FDH

Figur 4

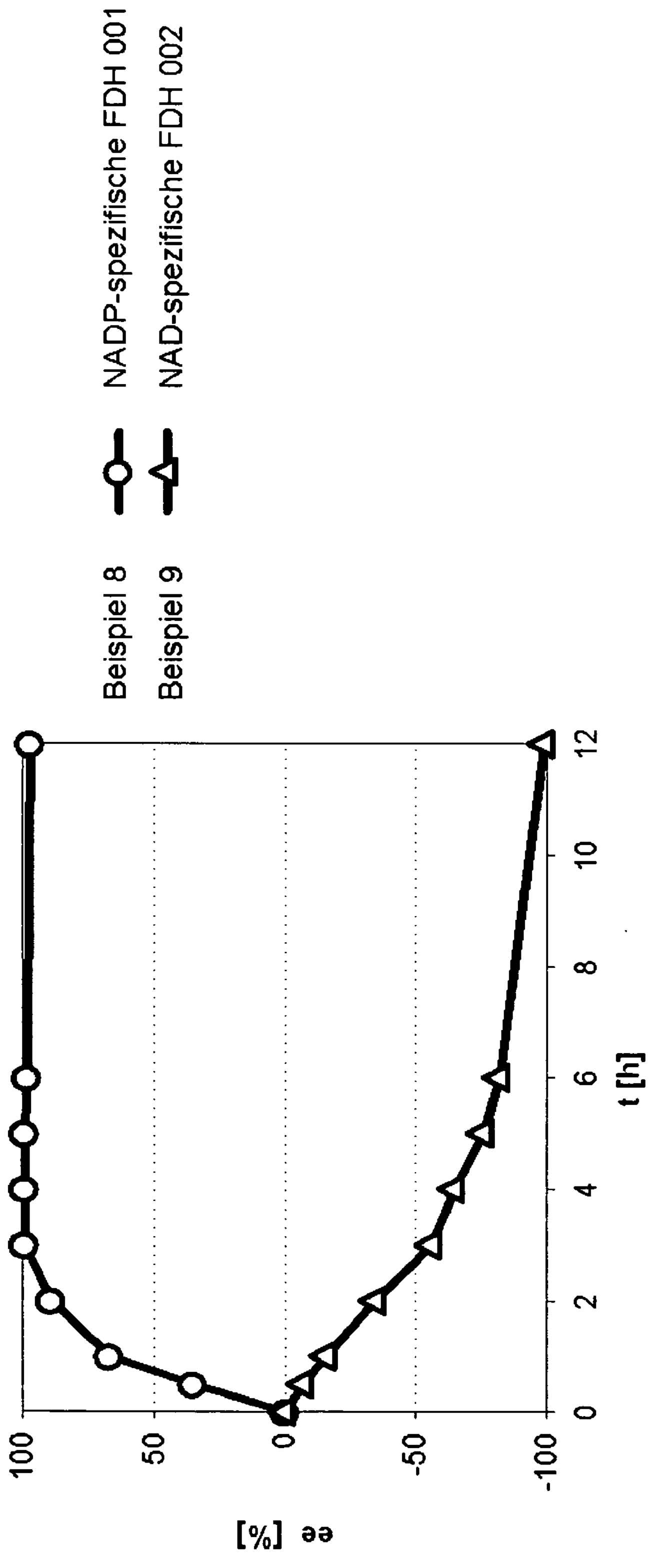
Beispiel 8
NADP-spezifische FDH 001
Beispiel 9
NAD-spezifische FDH 002
ee [%]
100
50
0
-50
-100
t [h]
0
2
4
6
8
10
12

Figur 5

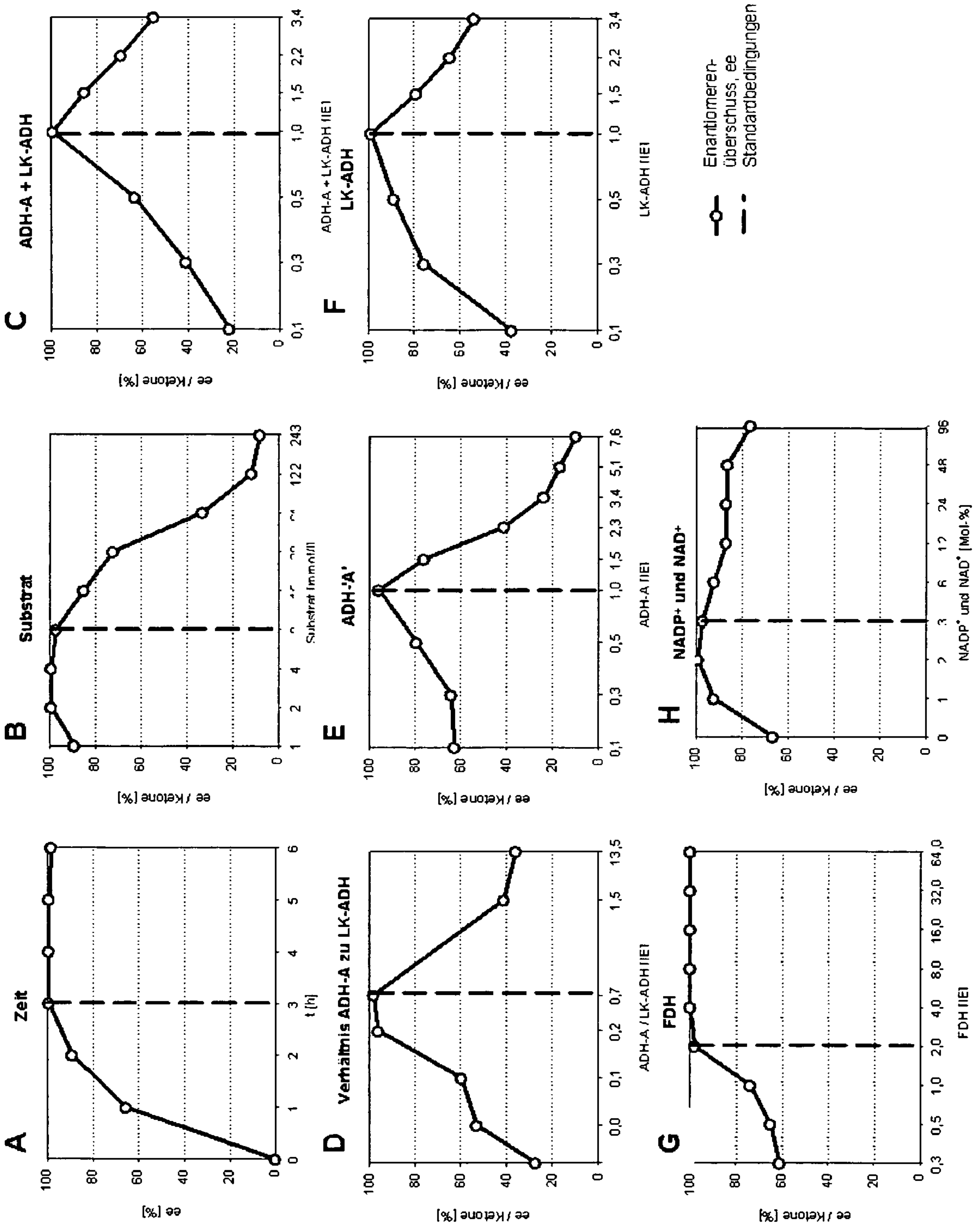
A
Zeit
ee [%]
t [h]
B
Substrat
ee / Ketone [%]
C
ADH-A + LK-ADH
ee / Ketone [%]
ADH-A + LK-ADH [E]
D
Verhältnis ADH-A zu LK-ADH
ee / Ketone [%]
ADH-A / LK-ADH [E]
E
ADH-'A'
ee / Ketone [%]
ADH-A [E]
F
LK-ADH
ee / Ketone [%]
LK-ADH [E]
G
FDH
ee / Ketone [%]
FDH [E]
H
NADP+ und NAD+
ee / Ketone [%]
NADP+ und NAD+ [Mol-%]
Enantiomeren-
überschuss, ee
Standardbedingungen

Figur 6

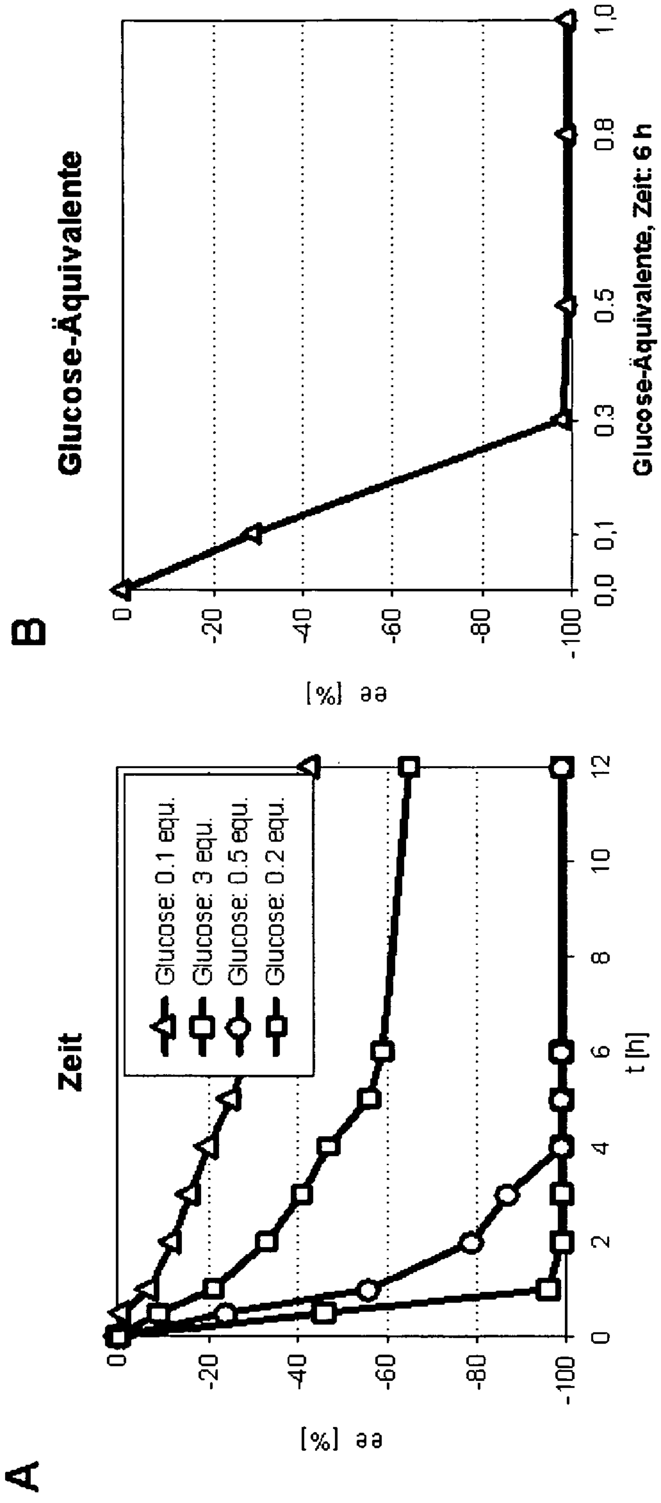
A
Zeit
ee [%]
t [h]
Glucose: 0.1 equ.
Glucose: 3 equ.
Glucose: 0.5 equ.
Glucose: 0.2 equ.
B
Glucose-Äquivalente
ee [%]
Glucose-Äquivalente, Zeit: 6 h

Figur 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1854893 A1


### In der Beschreibung aufgeführte Nicht-Patentliteratur


- **O. Pamies ; J.-E. Bäckvall.** *Trends Biotechnol.,* 2004, vol. 22, 130-135
- *Chem. Rev.,* 2003, vol. 103, 3247-3261
- **H. Pellissier.** *Tetrahedron,* 2003, vol. 59, 8291-8327
- **M. J. Kim ; Y. Ahn ; J. Park.** *Curr. Opin. Biotechnol.,* 2002, vol. 13, 578-587
- **V. Zimmermann ; M. Beller ; U. Kragl.** *Org. Process Res. Dev.,* 2006, vol. 10, 622-627
- **Y. Asano ; S. Yamaguchi.** *J. Am. Chem. Soc.,* 2005, vol. 127, 7696-7697
- **B. Schnell ; K. Faber ; W. Kroutil.** *Adv. Synth. Catal.,* 2003, vol. 345, 653-666
- *Chem. Eur. J.,* 2007, vol. 13, 8271-8276
- **C. V. Voss ; C. C. Gruber ; W. Kroutil.** *Angew. Chem. Int. Ed.,* 2008, vol. 47, 741-745